## Europäisches Patentamt

## European Patent Office

## Office européen des brevets

(19)

(11) Veröffentlichungsnummer : **0 019 789**
**B1**

(12) **EUROPÄISCHE PATENTSCHRIFT**

(45) Veröffentlichungstag der Patentschrift :
22.09.82

(51) Int. Cl.³ : **C 07 C 25/10, C 07 C 17/22**

(21) Anmeldenummer : **80102622.0**

(22) Anmeldetag : **12.05.80**

(54) Verfahren zur Herstellung von 1,3,5-Trichlorbenzol.

(30) Priorität : **18.05.79 DE 2920173**

(43) Veröffentlichungstag der Anmeldung :
**10.12.80 (Patentblatt 80/25)**

(45) Bekanntmachung des Hinweises auf die Patenterteilung : **22.09.82 Patentblatt 82/38**

(84) Benannte Vertragsstaaten :
**CH DE FR GB IT LI NL**

(56) Entgegenhaltungen :
**FR A 1 503 153**
**GB A 987 000**

**HOUBEN-WEYL : « Methoden der organischen Chemie », 4te Auflage, Band V/3, 1962, Georg Thieme Verlag, Halogenverbindungen Stuttgart, DE**

(73) Patentinhaber : **HOECHST AKTIENGESELLSCHAFT**
**Postfach 80 03 20**
**D-6230 Frankfurt/Main 80 (DE)**

(72) Erfinder : **Volkwein, Gert, Dr.**
**Im Schulzehnten 7**
**D-6233 Kelkheim (Taunus) (DE)**
Erfinder : **Baessler, Konrad, Dr.**
**Drosselweg 1**
**D-6230 Frankfurt am Main 80 (DE)**
Erfinder : **Wolfram, Hans, Dr.**
**Johann-Strauss-Strasse 40**
**D-6233 Kelkheim (Taunus) (DE)**

EP 0 019 789 B1

Imprimerie Jouve, 18, rue St-Denis, 75001 Paris, France

Verfahren zur Herstellung von 1,3,5-Trichlorbenzol

1,3,5-Trichlorbenzol ist ein wertvolles Zwischenprodukt auf verschiedenen Gebieten, z.B. auf dem Pflanzenschutzsektor zur Herstellung u.a. des Pflanzenschutzmittels Trichlor-trinitrobenzol.

Durch direkte Chlorierung von Benzol ist 1,3,5-Trichlorbenzol nicht zugänglich ; es muß daher auf anderen Wegen synthetisiert werden.

So ist es bekannt, 1,2,4-Trichlorbenzol durch Isomerisierung mit Friedel-Crafts-Katalysatoren und Cokatalysatoren wie Magnesiumsulfat, Methanol oder Wasser in 1,3,5-Trichlorbenzol zu überführen [US-PS 2 866 829 ; Z. obšc. Chim. 34 (1964) 1, 237 ; DE-PS 947 304 (1956)].

Diese Verfahren sind jedoch alle wegen relativ schlechtem Umsatz und anschließendem erheblichen Fraktionieraufwand ziemlich unbefriedigend.

Andere Synthesemethoden verlaufen über mehrere oft recht aufwendig durchzuführende Reaktionsstufen wie Diazotierungen und Verkochungen, z.B. des 2,4,6-Trichloranilins [J. of Chem. Soc. (London) (1947), 173] oder des schwer zugänglichen 3,5-Dichloranilins [Rec. Trav. Chim. d. Pays-Bas 37 (1918), 198].

Es ist weiterhin bekannt [Houben-Weyl Bd. V/3 (1962), S. 750], daß bei erhöhter Temperatur in Abwesenheit von Katalysatoren, welche die Kernchlorierung bewirken, durch Einwirkung von elementarem Chlor Nitrogruppen durch Chlor ersetzt werden können (« denitrierende Chlorierung »). Dabei werden unterschiedlich eine oder zwei Nitrogruppen ausgetauscht ; eine Verschiebung der Substituenten tritt nicht ein. So wird aus m-Dinitrobenzol bei 220 °C in 79 % d.Th. Ausbeute ein Gemisch aus 68 % m-Dichlorbenzol und 25 % m-Chlornitrobenzol erhalten (BIOS, Final Rep. 986 I, 151) ; ähnlich verläuft die Reaktion bei o-Dinitrobenzol, während beim p-Dinitrobenzol nur eine Nitrogruppe ausgetauscht wird und p-Chlornitrobenzol entsteht [Ber. deutsch. Chem. Ges. 24 (1891), 3749]. Analog wird aus 2,4-Dinitrotoluol nur eine Nitrogruppe ausgetauscht und — unter gleichzeitiger Oxydation der CH3-Gruppe — Chlornitrobenzoesäure erhalten [Zh. Org. Khim. 9 (1973) 4, 762].

In dem Bestreben, die bekannten Verfahren zur Herstellung von 1,3,5-Trichlorbenzol zu verbessern oder ein neues verbessertes Verfahren zur Verfügung zu stellen, wurde nun gefunden, daß sich diese Aufgabe in einfacher und befriedigender Weise dadurch lösen läßt, daß man 5-Chlor-1,3-dinitrobenzol und/oder 3,5-Dichlornitrobenzol bei Temperaturen von etwa 200 bis 270 °C, vorzugsweise von etwa 220 bis 250 °C, mit Chlor behandelt.

Bei dieser Chlorbehandlung werden die Nitrogruppen der beiden genannten Ausgangsmaterialien glatt durch Chlor ausgetauscht ; die Reaktion stellt also eine denitrierende Chlorierung dar.

Der glatte Verlauf dieser denitrierenden Chlorierung war aufgrund des einschlägigen Standes der Technik außerordentlich überraschend ; denn bei der Chlorbehandlung der Dinitrobenzole und des 2,4-Dinitrotoluols wird nur eine Nitrogruppe glatt durch Chlor ausgetauscht und der Austausch der zweiten Nitrogruppe — wenn der Benzolkern also bereits durch Chlor substituiert ist — soll nur unvollständig (Bios, a.a.O. : m-Dinitrobenzol ⟶ Gemisch aus m-Dichlorbenzol + m-Chlornitrobenzol ; Ber. d. deutsch. chem. Ges. 24 a.a.O. :

o-Dinitrobenzol ⟶ Gemisch aus o-Dichlorbenzol + o-Chlornitrobenzol) oder überhaupt nicht gelingen (Berichte der deutschen chemischen Gesellschaft 24 a.a.O. : p-Dinitrobenzol ⟶ p-Chlornitrobenzol ; Zh. Org. Chim. 9 a.a.O. : 2,4-Dinitrotoluol ⟶ Chlornitrobenzoesäure).

Das Verfahren wird zweckmäßig so durchgeführt, daß in das vorgelegte, von Kernchlorierungskatalysatoren freie — insbesondere eisenfreie — Ausgangsprodukt (5-Cl-1,3-Dinitrobenzol oder 3,5-Dichlornitrobenzol oder eine beliebige Mischung aus beiden Verbindungen) bei etwa 200-270 °C, vorzugsweise etwa 230 bis 250 °C, so lange Chlor eingeleitet wird, bis das entstehende Trichlorbenzol am Rückfluß kocht ; die bevorzugte Chlormenge muß molzahlmäßig mindestens gleich der Hälfte der Zahl der Nitrogruppen/Molekül der Ausgangsverbindung sein. Wenn also etwa 1 Mol 5-Cl-1,3-Dinitrobenzol als Ausgangsverbindung verwendet wird, muß mit mindestens 1 Mol Chlor chloriert werden ; im Falle der Verwendung von 3,5-Dichlornitrobenzol als Ausgangsprodukt ist der Einsatz von mindestens 0,5 Mol Chlor/Mol Ausgangsprodukt erforderlich. Geht man von einer Mischung der beiden genannten Ausgangsverbindungen aus, so ist eine entsprechende zwischen diesen Chlormengen liegende Mindestchlormenge nötig. Bei gleichbleibender Chlorbelastung wird sodann über eine zwischengeschaltete Kolonne das praktisch nitroproduktfreie Trichlorbenzol abdestilliert und gleichzeitig das zu denitrierende Produkt in den Sumpf zugegeben, so daß immer eine konstante Menge im Sumpf vorhanden ist. Diese kontinuierliche Fahrweise erlaubt die Verwendung relativ kleiner Reaktionsgefäße und liefert abhängig von der Länge der verwendeten Kolonne 1,3,5-Trichlorbenzol mit einer Reinheit ≧ 99 % in einer Ausbeute von etwa 95 % d.Th ; die kontinuierliche Verfahrensweise ist daher gegenüber der diskontinuierlichen bevorzugt. Das anfallende Reaktionsprodukt muß dann nur neutral gewaschen und getrocknet werden.

In einer weiteren bevorzugten Ausführungsform des erfindungsgemäßen Verfahrens wird das Ausgangsprodukt 5-Chlor-1,3-dinitrobenzol im Gemisch mit m-Dinitrobenzol eingesetzt. Diese Ausführungsform ist deswegen bevorzugt, weil das Gemisch aus 5-Chlor-1,3-dinitrobenzol und m-Di-nitrobenzol bei der (unvollständigen) Kernchlorierung von m-Dinitrobenzol anfällt und nach entsprechender Reinigung (Abtrennung vom Kernchlorierungskatalysator) direkt für die erfindungsgemäße Reaktion eingesetzt werden kann. Das Gemisch kann im Prinzip beliebig zusammengesetzt sein — die Zusammensetzung hängt vom Chlorierungsgrad des m-Dinitrobenzols ab, doch soll der Anteil an 5-Chlor-1,3-dinitrobenzol vorteilhaft mindestens etwa 20 Gew.%, insbesondere mindestens etwa 40 Gew.%, betragen. Bei der Chlorierung mit der zum vollständigen Austausch aller Nitrogruppen ausreichenden Chlormenge entsteht dann ein Gemisch aus 1,3,5-Trichlorbenzol und m-Dichlorbenzol. Der vollständige Austausch der Nitrogruppen auch des m-Dinitrobenzols durch Chlor war im Hinblick auf Bios a.a.O. überraschend ; möglicherweise übt aber der Austausch der Nitrogruppen des 5-Chlor-1,3-dinitrobenzols einen gewissen induzierenden Effekt auch auf den vollständigen Austausch der Nitrogruppen des m-Dinitrobenzols aus.

Die denitrierende Chlorierung des 5-Chlor-1,3-dinitrobenzols im Gemisch mit m-Dinitrobenzol läßt sich — ebenso wie die erfindungsgemäße Chlorbehandlung des 5-Chlor-1,3-dinitrobenzols alleine oder im Gemisch mit 3,5-Dichlornitrobenzol — sowohl diskontinuierlich als auch kontinuierlich durchführen, wobei die kontinuierliche Durchführungsweise wiederum bevorzugt ist. Die Siedepunktsunterschiede der Reaktionsprodukte 1,3,5-Trichlorbenzol und m-Dichlorbenzol stellen auch für die kontinuierliche Durchführungsweise kein Hindernis dar. Wegen dieses Siedepunktsunterschieds ist die Trennung des 1,3,5-Trichlorbenzols vom m-Dichlorbenzol leicht (destillativ) möglich.

Das erfindungsgemäße Verfahren liefert in wirtschaftlicher und nicht umweltschädigender Weise 1,3,5-Trichlorbenzol (und gegebenenfalls auch m-Dichlorbenzol) in guter bis sehr guter Ausbeute auch im technischen Maßstab und stellt daher einen erheblichen Fortschritt dar.

Die folgenden Beispiele sollen nun der weiteren Erläuterung der Erfindung dienen.

Beispiel 1

In einem 2 1-Kolben mit Gaseinleitungsrohr, aufgesetzter gut isolierter Vakuummantelkolonne mit ca. 20 theoretischen Böden und Rücklaufteiler und mit einer Heizung durch eine Pilzheizhaube, werden 1 500 g 3,5-Dichlornitrobenzol vorgelegt und auf 230 °C aufgeheizt. Dann beginnt man mit einer Belastung von 12 l/Stunde Chlor einzuleiten. Nach ca. 5 Stunden hat sich soviel 1,3,5-Trichlorbenzol gebildet, daß in der Fraktionierkolonne ein Rücklauf erzeugt wird. Im Sumpf ist dann ein Roh-EP. von ca. 54 °C erreicht worden, was einem Gehalt von ca. 15 % 1,3,5-Trichlorbenzol entspricht.

Man beginnt dann mit der Abnahme des 1,3,5-Trichlorbenzols und läßt in gleichem Maße 3,5-Dichlornitrobenzol geschmolzen so zulaufen, daß die Füllung im Sumpf konstant bleibt. Bei einem Zulauf von 150 g = ca. 110 ccm (80 °C) 3,5-Dichlornitrobenzol pro Stunde erhält man stündlich ca. 140 g = 105 ccm (80 °C) 1,3,5-Trichlorbenzol roh, das mit Wasser und verdünnter Natronlauge gewaschen und anschließend bei 100 °C/50 mm geschmolzen getrocknet wird. Pro Stunde fallen 134 g = ca. 100 ccm (80 °C) 1,3,5-Trichlorbenzol, rein, = 94,6 % d.Th., mit EP. 62,4 °C an.

GC-Analyse :

    0,2 % Dichlorbenzole
    99,5 % 1,3,5-Trichlorbenzol
    0,2 % 3,5-Dichlornitrobenzol
    0,1 % mehrere unbekannte Substanzen

Beispiel 2

In einem 2 1-Kolben mit Gaseinleitungsrohr und aufgesetzter gut isolierter Kolonne mit ca. 6 theoretischen Böden und Rücklaufteiler (beheizbar) und mit einer Heizung durch eine Pilzheizhaube, werden 1 200 g Chlorierungsgemisch (54 % m-Dinitrobenzol, 40 % 5-Chlor-1,3-dinitrobenzol) vorgelegt und auf 230 °C aufgeheizt. Dann beginnt man mit einer Belastung von 20 l/Stunde Chlor einzuleiten. Nach ca. 8 Stunden haben sich im Sumpf so viel leicht siedende Produkte gebildet, daß das Reaktionsgemisch siedet und in der Fraktionierkolonne ein Rücklauf erzeugt wird. Die Chlorbelastung wird dann auf 15 l/Stunde gesenkt und man beginnt mit der Abnahme von Trichlorbenzol/Dichlorbenzol-Gemisch und läßt im gleichen Maße Chlorierungsgemisch zulaufen, so daß die Füllung im Sumpf immer konstant bleibt.

In den ersten 6 bis 8 Stunden werden Produkte erhalten, die 80 bis 60 % m-Dichlorbenzol enthalten, da dessen Gehalt im Sumpf erst auf 2 bis 3 % absinkt und der Gehalt an 1,3,5-Trichlorbenzol auf 15 bis 20 % ansteigt. Dann werden konstante Bedingungen erreicht mit einer Kopftemperatur von ca. 170 °C.

Bei einem Zulauf von 80 g-ca. 57 ccm (80 °C) Chlorierungsgemisch pro Stunde erhält man stündlich ca. 70 g-55 ccm (80 °C) 1,3,5-Trichlorbenzol/m-Dichlorbenzol-Gemisch, roh, das mit Wasser und verdünnter Natronlauge neutralgewaschen und anschließend geschmolzen getrocknet wird. Pro Stunde fallen 66 g = ca. 50 ccm (50 °C) 1,3,5-Trichlorbenzol/m-Dichlorbenzol-Gemisch, = 82,5 % d.E., = ca. 93 % d.Th., mit EP. 17,5 °C an.

GC-Analyse :

    54   % m-Dichlorbenzol
    0,3 % o-Dichlorbenzol
    40   % 1,3,5-Trichlorbenzol
    3   % 1,2,4-Trichlorbenzol
    0,3 % 1,2,3-Trichlorbenzol
    0,2 % Tetrachlorbenzole
    2   % m-Chlornitrobenzol

0,2 % 3,5-Dichlornitrobenzol

Die Isolierung von 1,3,5-Trichlorbenzol und m-Dichlorbenzol erfolgt durch fraktionierte Destillation in bekannter Weise.

**Ansprüche**

1. Verfahren zur Herstellung von 1,3,5-Trichlorbenzol, dadurch gekennzeichnet, daß man 5-Chlor-1,3-dinitrobenzol und/oder 3,5-Dichlornitrobenzol bei Temperaturen von etwa 200 bis 270 °C, vorzugsweise von etwa 230 bis 250 °C, mit Chlor behandelt.

2. Verfahren nach Anspruch 1, dadurch gekennzeichnet, daß man das Verfahren kontinuierlich durchführt.

3. Verfahren nach den Ansprüchen 1 bis 2, dadurch gekennzeichnet, daß man das Ausgangsmaterial 5-Chlor-1,3-dinitrobenzol im Gemisch mit m-Dinitrobenzol einsetzt.

**Claims**

1. A process for the manufacture of 1,3,5-trichlorobenzene, characterized in treating 5-chloro-1,3-dinitrobenzene and/or 3,5-dichloronitrobenzene with chlorine at a temperature from about 200 to 270 °C, preferably from about 230 to 250 °C.

2. The process as claimed in claim 1, characterized by being carried out continuously.

3. The process as claimed in claims 1 to 2, characterized in using a starting product consisting 5-chloro-1,3-dinitrobenzene and of m-dinitrobenzene.

**Revendications**

1. Procédé pour préparer le trichloro-1,3,5 benzène, caractérisé en ce qu'on traite le chloro-5 dinitro-1,3 benzène et/ou le dichloro-3,5 nitrobenzène par du chlore à des températures d'environ 200 à 270 °C, de préférence d'environ 230 à 250 °C.

2. Procédé selon la revendication 1, caractérisé en ce qu'on effectue le procédé en continu.

3. Procédé selon l'une des revendications 1 et 2, caractérisé en ce qu'on utilise, comme matière de départ, du chloro-5 dinitro-1,3 benzène en mélange avec du m-dinitro-benzène.